# EUROPEAN PATENT SPECIFICATION

(11) **EP 2 759 979 B1**
(45) Date of publication and mention of the grant of the patent: **17.08.2022**
(21) Application number: 12833660.9
(22) Date of filing: 20.09.2012
(51) Int. Cl.: G16H 20/17, G16H 70/40

(54) **DRUG INFORMATION MANAGEMENT DEVICE AND DRUG INFORMATION MANAGEMENT METHOD**
VORRICHTUNG ZUR VERWALTUNG VON ARZNEIMITTELINFORMATIONEN UND VERFAHREN ZUR VERWALTUNG VON ARZNEIMITTELINFORMATIONEN
DISPOSITIF DE GESTION D'INFORMATIONS SUR DES MÉDICAMENTS ET PROCÉDÉ DE GESTION D'INFORMATIONS SUR DES MÉDICAMENTS

(30) Priority: 22.09.2011 JP 2011207729
(43) Date of publication of application: 30.07.2014
(73) Proprietor: Terumo Kabushiki Kaisha, Tokyo 151-0072 (JP)
(72) Inventor: ASAMA, Koichiro, Ashigarakami-gun Kanagawa 259-0151 (JP); TOUNOOKA, Hiroko, Kanagawa 257-0014 (JP)
(74) Representative: Casalonga
(86) International application number: PCT/JP2012/005989
(87) International publication number: WO 2013/042370

(56) References cited:
- WO-A2-2005/055112
- WO-A2-2005/066872
- BRELAND BURNIS D: "Continuous quality improvement using intelligent infusion pump data analysis", AMERICAN JOURNAL OF HEALTH-SYSTEM PHARMACY,, vol. 67, no. 17, 1 September 2010 (2010-09-01), pages 1446-1455, XP009184311, ISSN: 1079-2082, DOI: 10.2146/AJHP090588
- VANDERVEEN T W: "Using Data to Improve Smart Intravenous Infusion Pumps", BIOMEDICAL INSTRUMENTATION & TECHNOLOGY, ASSOCIATION FOR THE ADVANCEMENT OF MEDICAL INSTRUMENTATION, US, vol. 44, no. 6, 1 November 2010 (2010-11-01), pages 57-63, XP009184301, ISSN: 0899-8205, DOI: 10.2345/0899-8205-44.S1.57
- ESKEW J A ET AL: "Using innovative technologies to set new safety standards for the infusion of intravenous medications", HOSPITAL PHARMACY, LIPPINCOTT, PHILADELPHIA, US, vol. 37, no. 11, 1 November 2002 (2002-11-01), pages 1179-1189, XP002344385, ISSN: 0018-5787
- "PROCEEDINGS FROM THE ISMP SUMMIT ON THE USE OF SMART INFUSION PUMPS: GUIDELINES FOR SAFE IMPLEMENTATION AND USE", , 1 January 2009 (2009-01-01), XP055190583, Retrieved from the Internet: URL:https://www.ismp.org/Tools/guidelines/ smartpumps/comments/printerVersion.pdf [retrieved on 2015-05-20]

## Description

### Technical Field

The present invention relates to a medicine information management device and a medicine information management method that manage medicine information such as information on use of medicines used in medical equipment such as an infusion pump, for example.

### Background Art

In the related art, since there is a possibility that medicines used in medical equipment such as an infusion pump used in the hospital or the like cause some problems depending on the usage, there is a case where a limitation is imposed on, for example, the medicine dosage.

Moreover, when a medicine on which a limitation is imposed in this way is used, a configuration is adopted in which a limitation (limit) is defined in advance in medicine information, it is determined whether the usage is within the setting range with reference to the medicine dosage or the like, and, in a case where it is outside the setting range, an alert or the like is sounded (for example, Patent Literature 1).

### Citation List

### Patent Literature

Patent Literature 1: WO 2004/072828 A. Document BRELAND BURNIS D: "Continuous quality improvement using intelligent infusion pump data analysis",AMERICAN JOURNAL OF HEALTH-SYSTEM PHARMACY,, vol. 67, no. 17, 1 September 2010 (2010-09-01), pages 1446-1455, relates to an infusion pump which can modify soft limits, however, such modification only occurs in case of calculation or typing errors and not upon analysis of the data. This document is considered the closest prior art.

### Summary of Invention

### Technical Problem

However, such limitation (limit) on the use of the medicine that was uniformly defined is the one determined to be optimal in a certain period, and, with the advancement of medicine or the like, there is a problem that it is not necessarily correct.

Therefore, it is an object of the present invention to achieve a medicine information management device and a medicine information management method that enable CQI (Continuous Quality Improvement) of information on the use limitation or the like of medicines used in medical equipment or the like. Solution to Problem

In the present invention, the above-mentioned object is achieved by a medicine information management device including: generating and storing administration-level-information-associated information on the number of doses that is actual information on the number of doses of a medicine in multiple items of administration level information with respect to a subject of the medicine used in medical equipment; a medicine limitation information storage unit configured to store medicine limitation information that is limitation information for limiting a use of medicine information of the medicine in association with the administration level information; storing trouble information generated in a case where the medicine is actually used in the administration level information, in association with the medicine information and the administration level information; generating and storing number-of-doses-and-level-associated information on the number and level of troubles based on number information of the trouble information and the administration-level-information-associated information on the number of doses; generating medicine analysis information to show at least the number-of-doses-and-level-associated information on the number and level of troubles and the medicine limitation information, as information associated with the administration-level-information-associated information on the number of doses; and displaying this medicine analysis information on a display unit of a target side terminal.

According to the configuration, there is provided a configuration in which medicine analysis information to show the number-of-doses-and-level-associated information on the number and level of troubles and the medicine limitation information is generated as information associated with the administration-level-information-associated information on the number of doses, and this medicine analysis information is displayed on the display unit of the target side terminal. Since this number-of-doses-and-level-associated information on the number and level of troubles is generated on the basis of the number information of the trouble information (for example, existence/nonexistence of an alert) and the administration-level-information-associated information on the number of doses (for example, solution transmission number data of each medicine administration rate), it is, for example, information on alert occurrence frequency or the like with respect to the solution transmission number at each medicine administration rate.

Moreover, the medicine limitation information is information that defines the range of limitation information (for example, soft limit (an alert is output according to use) and hard limit (an alert is output and operation is stopped according to use)) that limits medicine information of a medicine in association with administration level information (for example, the administration rate of the medicine).

Meanwhile, the administration-level-information-associated information on the number of doses is information on the number of doses (for example, the solution transmission number of a medicine) of actual medicine information in each administration level information (for example, administration rate data and so on) of medicine information.

Therefore, for example, the doctor or the like who visually perceives the medicine analysis information shown in the display unit can promptly understand the alert occurrence frequency of each administration rate data and understand the current range of medicine limitation information (soft limit and hard limit) based on the administration rate at the same time.

Further, for example, by simultaneously comparing the alert occurrence frequency of each administration rate data and the ranges of the soft limit and the hard limit or the like, that are medicine limitation information, on the screen of the display unit, it is possible to promptly determine whether the medicine limitation information is appropriate.

By this means, by using the medicine information management device of the configuration, it is possible to execute CQI (Continuous Quality Improvement) of information on use limitation or the like of a medicine used in medical equipment or the like.

Preferably, there is a feature in which: a standard administration level information storage unit configured to store standard administration level information that is administration level information serving as a standard of the medicine information is included; and the medication administration standard information is included in the medicine analysis information as information associated with the administration-level-information-associated information on the number of doses.

According to the configuration, the standard administration level information storage unit configured to store the standard administration level information that is the administration level information serving as a standard of the medicine information is included, and the medication administration standard information is included in the medicine analysis information as information associated with the administration-level-information-associated information on the number of doses.

Therefore, since the doctor or the like who visually perceives the medicine analysis information on the display unit can compare the standard administration level information and the administration-level-information-associated information on the number of doses on the screen at the same time, it is possible to promptly determine whether the standard administration level information is appropriate.

Preferably, a feature lies in a configuration in which medicine limitation change suggestion information that suggests a change of the medicine limitation information is generated based on the medicine analysis information, and this medicine limitation change suggestion information is displayed on the display unit of the subject side terminal.

According to the configuration, there is provided a configuration in which the medicine limitation change suggestion information that suggests a change of the medicine limitation information is generated based on the medicine analysis information, and this medicine limitation change suggestion information is displayed on the display unit of the subject side terminal

Therefore, the doctor or the like who visually perceives the medicine limitation change suggestion information on the display unit can promptly know the change method or the like of the medicine limitation information. Therefore, it is possible to update the medicine limitation information to more preferable information, and execute CQI (Continuous Quality Improvement) of information on use limitation or the like of a medicine used in medical equipment and so on.

Preferably, a feature lies in a configuration in which standard administration level change suggestion information that suggests a change of standard administration level information is generated based on the medicine analysis information, and this standard administration level change suggestion information is displayed on the display unit of the subject side terminal.

According to the configuration, there is provided a configuration in which the standard administration level change suggestion information that suggests a change of the standard administration level information is generated based on the medicine analysis information, and this standard administration level change suggestion information is displayed on the display unit of the subject side terminal.

Therefore, the doctor or the like who visually perceives the standard administration level change suggestion information on the display unit can promptly know the change method or the like of the standard administration level information.

Therefore, it is possible to update the standard administration level information to more preferable information, and execute CQI (Continuous Quality Improvement) of information such as the standard administration level information or the like of a medicine used in medical equipment and so on.

Preferably, a feature lies in a configuration in which the medicine limitation information is changed based on the medicine analysis information.

According to the configuration, there is provided a configuration in which the medicine limitation information is changed based on the medicine analysis information. Therefore, it is possible to update the medicine limitation information to more preferable information, and execute CQI (Continuous Quality Improvement) of information on use limitation or the like of a medicine used in medical equipment and so on.

Preferably, a feature lies in a configuration in which standard administration level information is changed based on the medicine analysis information.

According to the configuration, there is provided a configuration in which the standard administration level information is changed based on the medicine analysis information. Therefore, it is possible to update the standard administration level information to more preferable information, and execute CQI (Continuous Quality Improvement) of information of standard administration level information or the like of a medicine used in medical equipment and so on.

In the present invention, the above-mentioned object is achieved by a medicine information management method including: generating and storing administration-level-information-associated information on the number of doses that is actual information on the number of doses of a medicine in multiple items of administration level information with respect to a subject of the medicine used in medical equipment; a medicine limitation information storage unit configured to store medicine limitation information that is limitation information for limiting a use of medicine information of the medicine in association with the administration level information; storing trouble information generated in a case where the medicine information is actually used in the administration level information, in association with the medicine information and the administration level information; generating and storing number-of-doses-and-level-associated information on the number and level of troubles based on number information of the trouble information and the administration-level-information-associated information on the number of doses; generating medicine analysis information to show at least the number-of-doses-and-level-associated information on the number and level of troubles and the medicine limitation information as information associated with the administration-level-information-associated information on the number of doses; and displaying this medicine analysis information on a display unit of a target side terminal.

### Advantageous Effects of Invention

As described above, according to the present invention, it is possible to provide a medicine information management device and a medicine information management method that enable CQI (Continuous Quality Improvement) of information on the use limitation or the like of medicines used in medical equipment or the like.

### Brief Description of Drawings

Fig. 1 is a schematic view illustrating a pump CQI system according to a first embodiment including, for example, a management server that is a medicine information management device of the present invention.
Fig. 2 is a schematic block diagram illustrating a main configuration of the infusion pump in Fig. 1.
Fig. 3 is a schematic diagram illustrating a main configuration of the management server in Fig. 1.
Fig. 4 is a schematic block diagram illustrating what are included in various storage units in Fig. 3.
Fig. 5 is a schematic flowchart illustrating the main operation of a CQI system according to the present embodiment, and so on.
Fig. 6 is another schematic flowchart indicating the main operation of a CQI system according to the present embodiment, and so on.
Fig. 7 is a schematic explanatory diagram illustrating pump data stored in the pump data storage unit in Fig. 4.
Fig. 8 is a schematic explanatory diagram illustrating each-medicine soft/hard limit data in each medicine hard/soft limit data storage unit in Fig. 4.
Fig. 9 is a graph illustrating, for example, a medicine library analysis graph that is analysis data per medicine library.
Fig. 10 is a schematic block diagram illustrating the main configuration of a management server according to a second embodiment of the present invention.
Fig. 11 is a schematic flowchart illustrating the main operation of a management server of a CQI system according to the present embodiment, and so on.

### Description of Embodiments

In the following, preferable embodiments of this invention are described in detail with reference to the accompanying drawings, and so on.

Note that, since the embodiments described below are preferable embodiments of the present invention, technically-preferable various limitations are assigned.

### (First Embodiment)

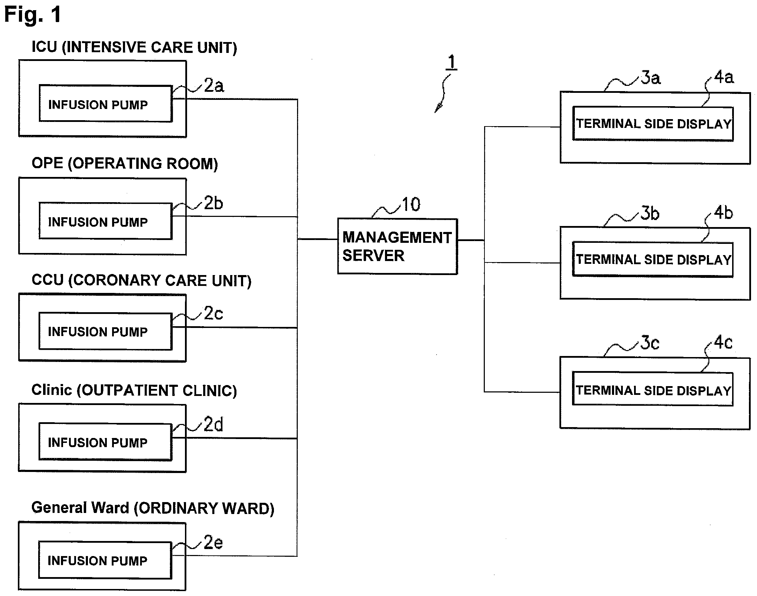

Fig. 1 is a schematic diagram illustrating a pump CQI (Continuous Quality Improvement) system 1 according to a first embodiment including, for example, a management server 10 that is a medicine information management device of the present invention.

As illustrated in Fig. 1, the pump CQI system 1 includes infusion pumps 2a to 2e arranged in an ICU (intensive care unit), OPE (operating room), CCU (coronary care unit), Clinic (outpatient clinic) and General Ward (ordinary ward), and so on, in the hospital.

These infusion pumps 2a and so on are medical equipment used together with an instrument, such as a drip, to accurately administer a medicine or the like to a patient.

Here, although an explanation is given using the infusion pump 2a or the like in the present embodiment, the medical equipment of the present invention may be a syringe pump or the like, besides the infusion pump 2a or the like.

As illustrated in Fig. 1, these infusion pumps 2a and so on are connected to the management server 10 such that communication with the server is possible. The infusion pumps are also connected to medical office terminals 3a to 3c, and so on, arranged in the medial office or the like of doctors and on the like, such that communication with the terminals is possible.

Here, in the medical office terminals 3a and so on, for example, terminal side displays 4a to 4c that are respectively display units are formed.

Also, in the present embodiment, for ease of explanation, an example is illustrated where the infusion pumps 2 and so on are directly connected to the management server 10 as illustrated in Fig. 1. However, the present invention is not limited to this, and a configuration is also possible in which each of the infusion pumps 2a and so on is connected to the management server through "software for pump communication (gateway terminal)" that manages each of the infusion pumps 2a and so on. In this case, the function of the management server 10 of the present embodiment is shared between the "software for pump communication (gateway terminal) " and the "management server".

The pump CQI system 1 of the present embodiment is a system to promote continuous quality improvement in medical treatment related to the infusion pumps 2a and so on. The system analyzes the use status or the like of medicines or the like from the operation history (pump history) collected from the infusion pumps 2a and so on, and intends to improve the method of use of the infusion pumps 2a and so on as well as the content of the medicine library (for example, data of medicine administration limitation or the like).

The infusion pumps 2a and so on, the management server 10 and the medical office terminals 4a and so on illustrated in Fig. 1 have a computer, the computer has an unillustrated CPU (Central Processing Unit), RAM (Random Access Memory) and ROM(Read Only Memory), and so on, and these are connected through a bus.

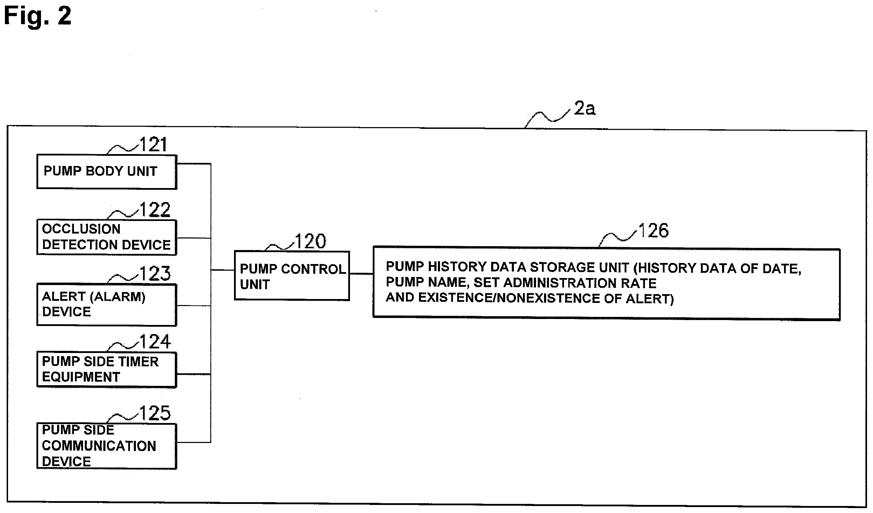

Fig. 2 is a schematic block diagram illustrating the main configuration of the infusion pumps 2a and so on in Fig. 1.

As illustrated in Fig. 2, the infusion pumps 2a and so on include a pump control unit 120, a pump body unit 121 that performs operation to administer a medicine for drip and the like, for example, a medical solution, to the patient at a predetermined administration rate (one example of administration level information), and an occlusion detection device 122 including a sensor that detects, in a case where the occlusion or the like occurs while the medical solution in the infusion pumps 2a and so on is administered, the fact.

Moreover, the infusion pumps 2a and so on include an alert (alarm) device 123 that informs, in a case where the occlusion of the infusion pumps 2a and so on is detected by the occlusion detection device 122, this trouble (one example of trouble information), pump side timer equipment 124 that is a clock or the like, and a pump side communication device 125 by which the infusion pumps 2a and so on perform communication with the management server 10, and so on.

The pump body unit 121 and so on is connected to the pump control unit 120 as illustrated in Fig. 1 and controlled.

Here, although a "pump history data storage unit" is also connected to the pump control unit 120 as illustrated in Fig. 1, this configuration is described later in detail.

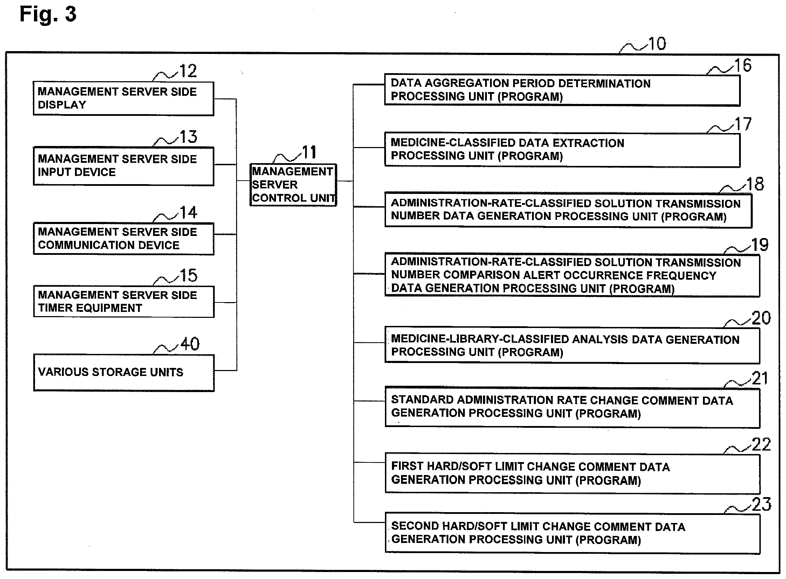

Fig. 3 is a schematic diagram illustrating the main configuration of the management server 10 in Fig. 1.

As illustrated in Fig. 3, the management server 10 includes a management server control unit 11, for example, a management server side display 12 that is a display unit to display various kinds of data, a management server side input device 13 that inputs various kinds of data, a management server side communication device 14 by which the management server 10 performs communication with the infusion pumps 2a and so on and the medical office terminals 3a and so on, and a management server side timer equipment 15 that is a clock or the like.

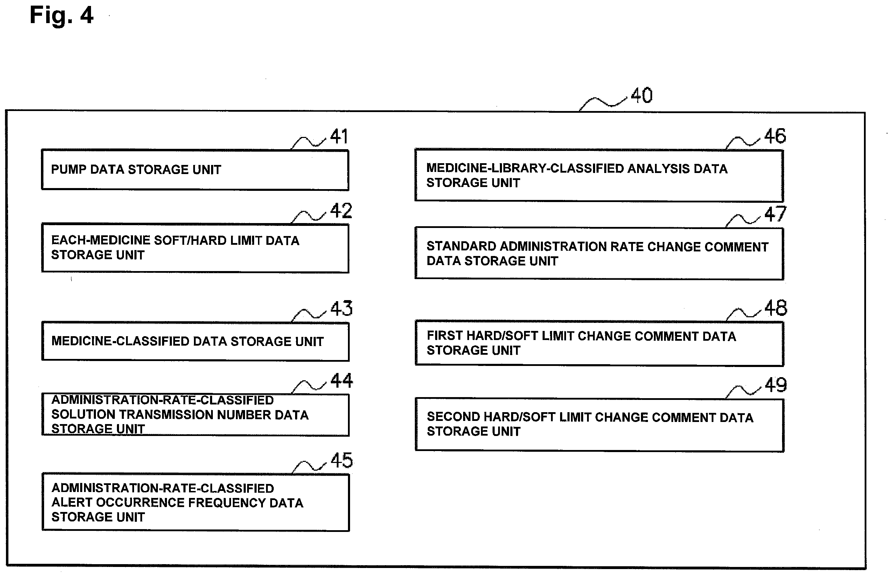

The management server side display 12 and so on are connected to the management server control unit 11 and controlled. Moreover, as illustrated in Fig. 3, the management server control unit 11 is configured to control various storage units 40 and various processing units (programs) . The content of these various storage units 40 and various processing units (programs) are described later in detail. Note that, although Fig. 4 is a schematic block diagram illustrating the content of the various storage units 40 in Fig. 3, the diagram is also described later in detail.

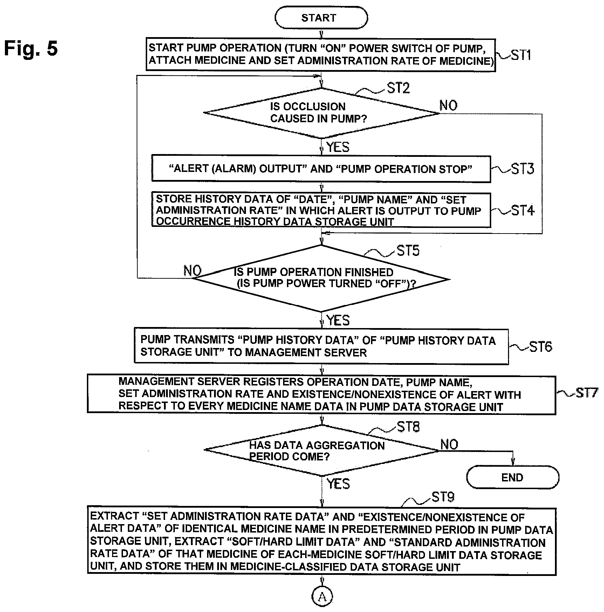

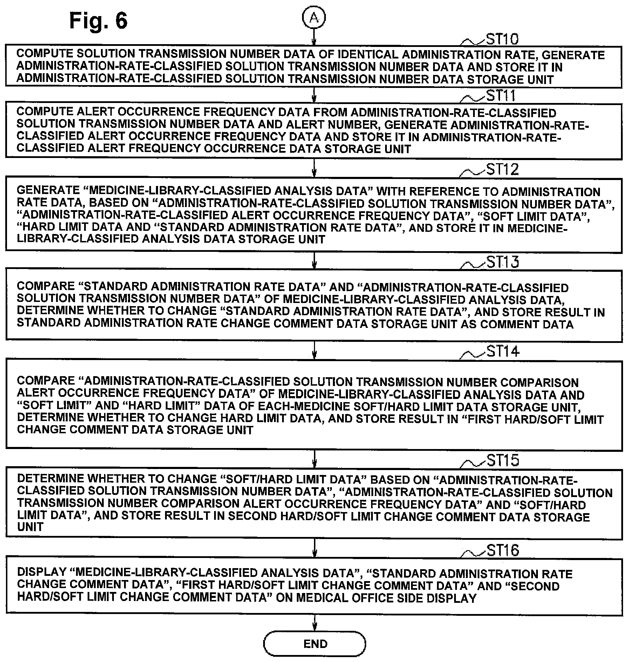

Figs. 5 and 6 are schematic flowcharts illustrating the main operation of the CQI system 1 according to the present embodiment, and so on. In the following, the present embodiment is described according to the flowcharts in Figs. 5 and 6, and the configurations in Figs. 1 to 4 are described.

First, in step (hereinafter referred to as "ST") 1 in Fig. 5, the power switch of the infusion pumps 2a and so on in Fig. 1 enters an "ON" state, and, for example, "Diprivan (registered trademark of AstraZeneca Ltd.)" that is a narcotic is attached to the infusion pumps 2a and so on, and the administration rate of medicine "Diprivan" with respect to the patient is set.

Next, it proceeds to ST2. In ST2, it is determined whether an occlusion is caused in the infusion pumps 2a and so on.

Specifically, the occlusion detection device 122 of the infusion pumps 2a and so on illustrated in Fig. 2 operates and determines whether the occlusion state is caused in the infusion pumps 2a and so on.

In a case where the occlusion is caused in the infusion pumps 2a and so on in ST2, it proceeds to ST3. In ST3, an alert (alarm) is output and the operation of the infusion pumps 2a and so on is stopped.

Specifically, the alert (alarm) device 124 in Fig. 2 operates to output an alert, and the pump body unit 121 operates to stop the pump operation.

Next, it proceeds to ST4. In ST4, history data as to "date" on which the alert is output, "pump name" of the infusion pumps 2a and so on, "set administration rate" of the medicine in the infusion pumps 2a and so on, and "existence/nonexistence of the alert", and on the like, are stored in the "pump history data storage unit 126" of the infusion pumps 2a and so on in Fig. 2. Moreover, regarding the "date", the pump side timer equipment 124 is referred to.

Next, it proceeds to ST5. In ST5, it is determined whether the pump operation has ended, and, in a case where it has, it proceeds to ST6.

In ST6, the infusion pumps 2a and so on use the pump side communication device 125 in Fig. 2 and transmit the "pump history data" stored in the pump history data storage unit 126 in pump history data 26 to the management server 10 in Fig. 1.

Next, it proceeds to ST7. In ST7, the management server 10 having received the data transmitted in ST6 stores the data in a pump data storage unit 41 in Fig. 4.

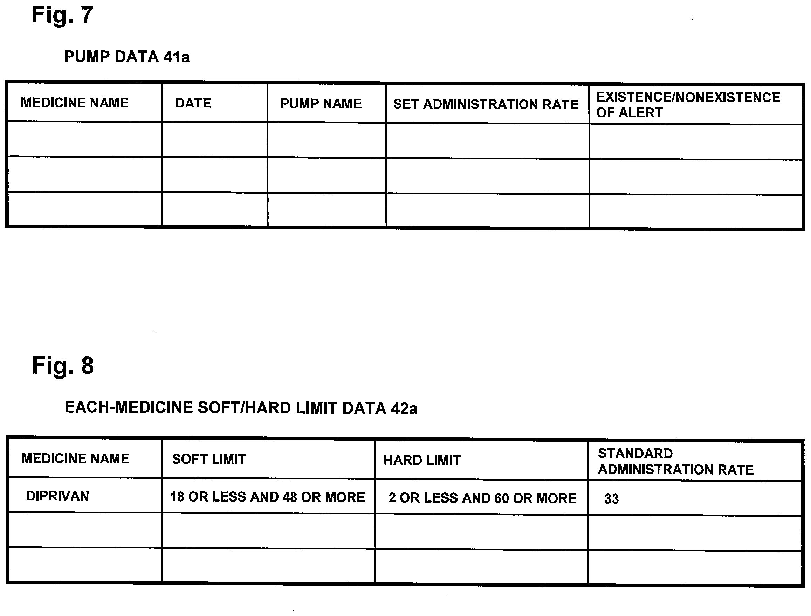

Fig. 7 is a schematic explanatory diagram indicating the "pump data 41a" stored in the pump data storage unit 41 in Fig. 4.

As illustrated in Fig. 7, for example, pieces of data, i.e. "medicine name", "date", "pump name", "set administration rate" and "existence/nonexistence of alert" that are medicine information are associated and stored in the pump data 41a.

The "Administration rate" is one example of administration level information and is shown in a unit of, for example, "ml/h" or the like. Moreover, the "alert" is one example of trouble information.

Next, it proceeds to ST8. In ST8, the management server 10 determines whether a data aggregation period has come. Specifically, a "data aggregation period determination processing unit (program) 16" in Fig. 3 operates, refers to the management server side timer equipment 15 in Fig. 3 and makes a determination.

In ST8, it proceeds to ST9 in a case where it is determined that the period has come. In ST9, a medicine-classified data extraction processing unit (program) 17 in Fig. 3 operates and extracts "set administration rate data" and "existence/nonexistence of alert data" of the identical medicine name (for example, "Diprivan") in a predetermined period in the pump data storage unit 41.

Moreover, the medicine-classified data extraction processing unit (program) 17 extracts "soft/hard limit data" and "standard administration rate" of that medicine "Diprivan" from a "each-medicine soft/hard limit data storage unit 42" in Fig. 4, and stores them in a "medicine-classified data storage unit 43" in Fig. 4.

Fig. 8 is a schematic explanatory diagram illustrating the "each-medicine soft/hard limit data 42a" in the "each-medicine soft/hard limit data storage unit 42" in Fig. 4.

As illustrated in Fig. 8, pieces of data, i.e. "soft limit", "hard limit" and "standard administration rate" are mutually associated with respect to every "medicine name" and registered in the "each-medicine soft/hard limit data 42a".

Here, the "soft limit" denotes limitation information on the administration rate when the medicine name "Diprivan" or the like is used in the infusion pumps 2a and so on, the medicine is not made to be set to the administration rate within the range of the soft limit, and the "alert (alarm)" is output regardless of the existence/nonexistence of actual generation of an occlusion or the like.

Meanwhile, the "hard limit" denotes limitation information on the administration rate when medicine "Diprivan" or the like is used in the infusion pumps 2a and so on, and has a configuration in which, when the medicine is set to the administration rate within the range of the hard limit, "alert (alarm)" is output regardless of the existence/nonexistence of actual generation of an occlusion or the like, and the operation of the infusion pumps 2a and so on is forcibly stopped.

Thus, by setting the range of the "soft limit" or the "hard limit" related to the administration rate different in each medicine beforehand, a configuration is provided to prevent an occlusion or the like of the infusion pumps 2a and so on caused by the medicine rate from being generated beforehand.

Moreover, the standard administration rate denotes the administration rate that is the standard of administration of the medicine, and, in the case of medicine "Diprivan" of the present embodiment, it is "33 ml/h" as illustrated in Fig. 8.

In the present embodiment, as illustrated in Fig. 8, the "soft limit" of the medicine name "Diprivan" is "18 ml/h or less and 48 ml/h or more" and the "hard limit" is "2 ml/h or less and 60 ml/h or more".

Thus, the "soft limit" and the "hard limit" are one example of the medicine limitation information, and the "each-medicine soft/hard limit data storage unit 42" is one example of the medicine limitation information storage unit.

Moreover, the "standard administration rate" in Fig. 8 is one example of the standard administration level information.

Next, it proceeds to ST10. In ST10, an "administration-rate-classified solution transmission number data generation processing unit (program) 18" in Fig. 3 operates, computes solution transmission number data of the identical administration rate, generates administration-rate-classified solution transmission number data and stores it in an administration-rate-classified solution transmission number data storage unit 44.

That is, data as to the number of times the medicine is sent at the identical administration rate is generated.

Therefore, the "solution transmission number" is one example of the information on the number of doses, and "administration-rate-classified solution transmission number data" is one example of the "information on the number of doses associated with administration level information".

Next, it proceeds to ST11. In ST11, an "administration-rate-classified solution transmission number comparison alert occurrence frequency data generation processing unit (program) 19" in Fig. 3 operates, generates "administration-rate-classified solution transmission number comparison alert occurrence frequency data" from the administration-rate-classified solution transmission number data in the administration-rate-classified solution transmission number data storage unit 44 and the "alert" number of the "existence/nonexistence" of the medicine-classified data storage unit 43, and stores it in an "administration-rate-classified solution transmission number comparison alert occurrence frequency data storage unit 35" in Fig. 4.

That is, the data of the "alert occurrence frequency" of each administration rate is generated from the solution transmission number data of each administration rate and the alert frequency data of each administration rate.

Thus, the "administration-rate-classified solution transmission number comparison alert occurrence frequency data" is one example of the "trouble number/level information associated with the number of doses and level".

Next, it proceeds to ST12. In ST12, a "medicine-library-classified analysis data generation processing unit (program) 20" in Fig. 3 operates, with reference to administration rate data, for example, generates "medicine-library-classified analysis data" that is medicine analysis information on the basis of "administration-rate-classified solution transmission number data" of the "administration-rate-classified solution transmission number data storage unit 44" in Fig. 4 (i.e., data as to the number of times a medicine is sent at the identical administration rate), "administration-rate-classified solution transmission number comparison alert occurrence frequency data" of an "administration-rate-classified solution transmission number comparison alert occurrence frequency data storage unit 45" (i.e., data of "alert occurrence frequency" of each administration rate), and the "soft limit data" (e.g., 18 ml/h or less and 48 ml/h or more)", the "hard limit data (2 ml/h or less and 60 ml/h or more) " and "standard administration rate data (33 ml/h) " of the "medicine-classified data storage unit 43", and stores it in a medicine-library-classified analysis data storage unit 46 in Fig. 4.

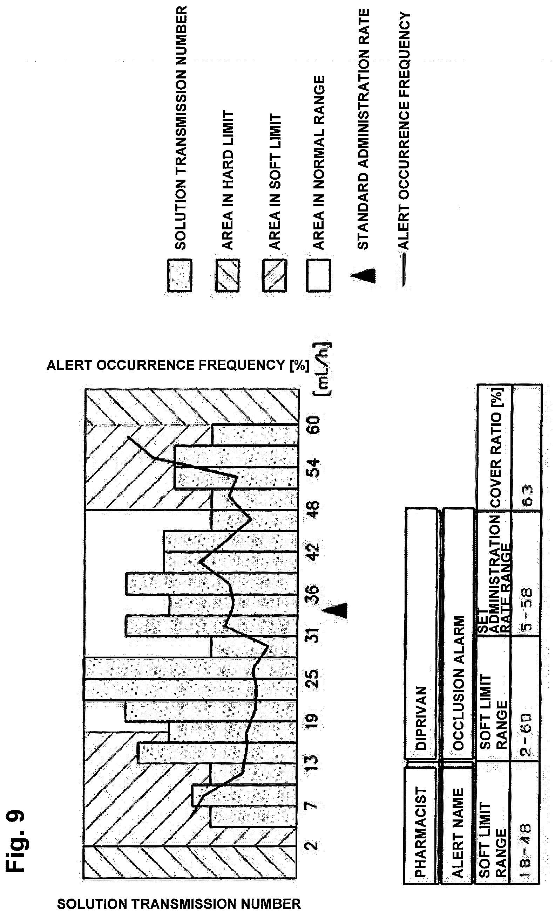

Fig. 9 is a graph illustrating, for example, a "medicine-library analysis graph" that is a medicine-library-classified analysis data. The graph is, for example, a medicine library analysis graph of medicine "Diprivan".

In the graph, "administration rate data" is arranged in the X axis direction, and the solution transmission number of each administration rate ("administration-rate-classified solution transmission number data") is illustrated by the bar chart in the Y axis direction.

Moreover, data of "alert occurrence frequency" of each administration rate ("administration-rate-classified solution transmission number comparison alert occurrence frequency data") is illustrated by the line graph.

Further, the ranges of "soft limit" and "hard limit" are displayed by diagonals.

Next, it proceeds to ST13. In ST13, a standard administration rate change comment data generation processing unit (program) 21 in Fig. 3 operates, compares the "standard administration rate data" and the "administration-rate-classified solution transmission number data" of the "medicine-library-classified analysis data" in the medicine-library-classified analysis data storage unit 46 in Fig. 4, determines whether to change the "standard administration rate data", and stores the result in a "standard administration rate change comment data storage unit 47" in Fig. 4 as "standard rate change comment data (one example of standard administration level change suggestion information)".

For example, referring to the "administration-rate-classified solution transmission number data" in a case where the current "standard administration rate data" is "33 ml/h" as illustrated in Fig. 9, when the number of doses is the largest at an administration rate of "25 ml/h" as illustrated in Fig. 9, in ST13, comment data to change the "standard administration rate data" from "33 ml/h" to "25 ml/h" is stored in the "standard administration rate change comment data storage unit 47" as "standard rate change comment data".

Next, it proceeds to ST14. In ST14, a "first hard/soft limit change comment data generation processing unit (program) 22" in Fig. 3 operates, compares the "administration-rate-classified solution transmission number comparison alert occurrence frequency data", the "soft limit" and the "hard limit" of the "medicine-library-classified analysis data storage unit 46" in Fig. 4, determines whether to change the hard limit data, and stores the result in a "first hard/soft limit change comment data storage unit 48" as "first hard/soft limit change comment data (one example of the medicine limitation change suggestion information)".

For example, in a case where the "administration-rate-classified solution transmission number comparison alert occurrence frequency data" in the soft limit is greater than a certain level as compared to other administration rates, the data serves as comment data to change the "hard limit data" such that, for example, "54 ml/h" in Fig. 9 which is the administration rate corresponding to that "administration-rate-classified solution transmission number comparison alert occurrence frequency data" is included in the "hard limit data".

Specifically, comment data to change and expand the range of the "hard limit" to the left side of Fig. 9 so as to include an administration rate of "54 ml/h" or more in the "hard limit" is generated and stored in the "first hard/soft limit change comment data storage unit 48".

This data is one example of the "first hard/soft limit change comment data".

Next, it proceeds to ST15. In ST15, a "second hard/soft limit change comment data generation processing unit (program) 23" in Fig. 3 operates, determines whether to change the "soft limit" data on the basis of the "administration-rate-classified solution transmission number data", the "administration-rate-classified solution transmission number comparison alert occurrence frequency data", the "soft limit" and the "hard limit" data of the "medicine-library-classified analysis data storage unit 46" in Fig. 4, and stores the result in a "second hard/soft limit change comment data storage unit 49" as "second hard/soft limit change comment data (one example of the medicine limitation change suggestion information)".

For example, In a case where the solution transmission number (frequency) of the "administration-rate-classified solution transmission number data" is greater than the others, the "administration-rate-classified solution transmission number comparison alert occurrence frequency data" is a low "administration rate", for example, "13 ml/h" in Fig. 9 and "13 ml/h" is included in the "soft limit" as illustrated in Fig. 9, comment data is generated to change the "soft limit" such that the "13 ml/h" is included in an "area within a normal range" instead of the soft limit. Specifically, it is comment data to change and reduce the range of the soft limit in Fig. 9 in the left direction in Fig. 9.

This data is one example of the "second hard/soft limit change comment data".

Next, it proceeds to ST16. In ST16, the management server 10 transmits the following data to the medical office terminals 3a and so on in Fig. 1.
1) "Medicine-library-classified analysis data (for example, data in Fig. 9)" in the medicine-library-classified analysis data storage unit 45 in Fig. 4
2) "Standard administration rate change comment data" of the standard administration rate change comment data storage unit 47 (for example, comment data to change "standard administration rate data" from "33 ml/h" to "25 ml/h")
3) "First hard/soft limit change comment data" in the first hard/soft limit change comment data storage unit 48 (for example, comment data to change and expand the range of the "hard limit" to the left side in Fig. 9 such that an administration rate of "54 ml/h") or more is included in the "hard limit").
4) "Second hard/soft limit change comment data" in the second hard/soft limit change comment data storage unit 49 (for example, comment data to change "soft limit" such that "13 ml/h" is included in the "area in the normal range" instead of the soft limit.

Further, the medical office terminals 3a and so on having received these items of data display these items of data on the terminal side displays 4a and so on of the terminals.

Therefore, by visually perceiving the "medicine-library-classified analysis data" which is illustrated in Fig. 9 and displayed on the terminal side displays 4a and so on, the doctor or the like in the medical office can promptly understand the alert occurrence frequency of each administration rate and, at the same time, immediately understand whether the ranges of the "soft limit" and the "hard limit" based on the administration rate are appropriate.

Moreover, the "standard administration rate change comment data" is also displayed on the terminal side displays 4a and so on together with the "medicine-library-classified analysis data".

Therefore, since the doctor or the like can promptly know the necessity of the change in the "standard administration rate data" and a specific change method on the basis of the "standard administration rate change comment data", it is possible to appropriately perform the change.

Moreover, the "first hard/soft limit change comment data" and the "second hard/soft limit change comment data" are also displayed on the terminal side displays 4a and so on together with the "medicine-library-classified analysis data".

Therefore, since the doctor or the like can specifically know the necessary of the change in the ranges of the "soft limit" and the "hard limit" and the change method, it is possible to appropriately perform the change.

As described above, according to the present embodiment, by using the management server 10, it is possible to achieve CQI of information on use limitation or the like of the "soft limit" and the "hard limit" or the like of the medicine used in the infusion pumps 2a and so on.

### (Second Embodiment)

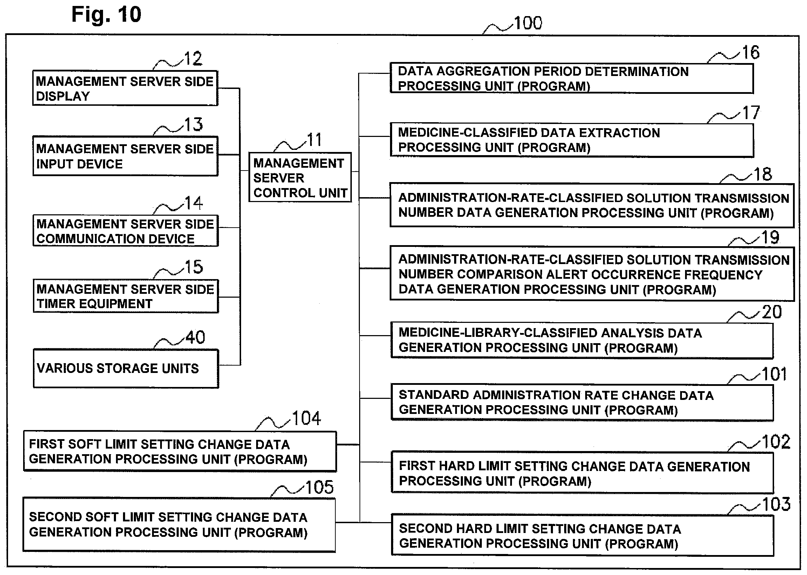

Fig. 10 is a schematic block diagram illustrating the main configuration of a management server 100 according to a second embodiment of the present invention.

Since the present embodiment has many common parts with the configuration of the first embodiment described above, the identical reference numerals or the like are assigned to the identical components or the like, explanation of the identical components is not repeated. In the following, differences are mostly described.

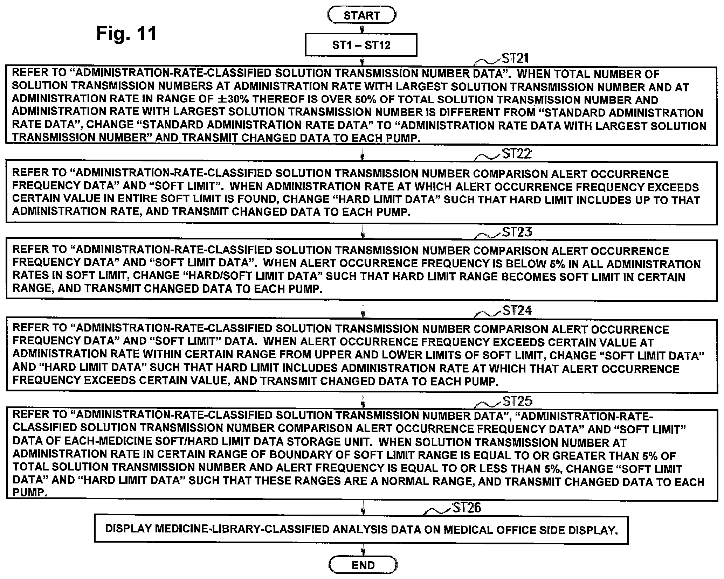

Fig. 11 is a schematic flowchart illustrating the main operation or the like of the management server 100 or the like of the CQI system according to the present embodiment.

First, in the present embodiment, similar to the first embodiment described above, steps ST1 to ST12 are executed and "medicine-library-classified analysis data" is generated and stored.

Next, it proceeds to ST21. In ST21, a "standard administration rate change data generation processing unit (program) 101" in Fig. 10 operates, refers to the "medicine-library-classified analysis data" in Fig. 4, and, in a case where the total number of the solution transmission numbers at the administration rate with the largest solution transmission number and at the administration rate in a range of ±30% thereof is over 50% of the total solution transmission number and the administration rate with the largest solution transmission number is different from "standard administration rate data" of the "medicine-library-classified analysis data storage unit 46", changes "standard administration rate data" of "medicine-library-classified analysis data" and an "each-medicine soft/hard limit data storage unit 42" or the like to the "administration rate data with the largest solution transmission number".

Moreover, the administration rate change data generation processing unit (program) 101 transmits this changed data to each of the infusion pumps 2a and so on in Fig. 1.

By this means, since the "standard administration rate data" of the medicine library data is always automatically updated as appropriate data, it is possible to execute a CQI process of medicine information used in the infusion pumps 2a and so on.

Next, it proceeds to ST22. In ST22, a "first hard limit setting change data generation processing unit (program) 102" in Fig. 10 operates, refers to "administration-rate-classified solution transmission number comparison alert occurrence frequency data" and "soft limit data" of the "medicine-library-classified analysis data", and, in a case where there is an "administration rate" at which the alert occurrence frequency exceeds a certain value in the entire administration rate in the soft limit, changes the "hard limit data" of the "medicine-library-classified analysis data" and the "each-medicine soft/hard limit data storage unit 42" or the like such that the "hard limit" includes up to that administration rate.

Moreover, the first hard limit setting change data generation processing unit (program) 102 transmits the changed data to each of the infusion pumps 2a and so on in Fig. 1.

Next, it proceeds to ST23. In ST23, a "second hard limit setting change data generation processing unit (program) 103" in Fig. 10 operates, refers to the "administration-rate-classified solution transmission number comparison alert occurrence frequency data" and the "soft limit data" of the "medicine-library-classified analysis data", and, when the alert occurrence frequency is below 5% in all administration rates in the soft limit, changes the "hard limit data" and the "soft limit data" of the "medicine-library-classified analysis data" and the "each-medicine soft/hard limit data storage unit 42" or the like so as to change the hard limit range to the soft limit range in a certain range (i.e., expand the soft limit range).

Moreover, the second hard limit setting change data generation processing unit (program) 103 transmits the changed data to each of the infusion pumps 2a and so on in Fig. 1.

Next, it proceeds to ST24. In ST24, a "first soft limit setting change data generation processing unit (program) 104" in Fig. 10 operates, refers to the "administration-rate-classified solution transmission number comparison alert occurrence frequency data" and the "soft limit data" of the "medicine-library-classified analysis data", refers to the "administration-rate-classified solution transmission number comparison alert occurrence frequency data" and the "soft limit data" of the "medicine-library-classified analysis data", and, in a case where the alert occurrence frequency exceeds a certain value at the administration rate within a certain range from the upper and lower limits of the soft limit, changes the "hard limit data" and the "soft limit data" of the "medicine-library-classified analysis data" and the "each-medicine soft/hard limit data storage unit 42" or the like such that the "hard limit" includes the administration rate at which the alert occurrence frequency exceeds the certain value.

Moreover, the first soft limit setting change data generation processing unit (program) 104 transmits the changed data to each of the infusion pumps 2a and so on in Fig. 1.

Next, it proceeds to ST25. In ST25, a "second soft the limit setting change data generation processing unit (program) 105" in Fig. 10 operates, refers to "administration-rate-classified solution transmission number data", "administration-rate-classified solution transmission number comparison alert occurrence frequency data" and the "soft limit data" of the "medicine-library-classified analysis data", and, in a case where the solution transmission number at the administration rate in a certain range of the boundary of the soft limit range is equal to or greater than 5% of the total solution transmission number and the alert frequency is equal to or less than 5%, changes the "hard limit data" and the "soft limit data" of the "medicine-library-classified analysis data" and the "each-medicine soft/hard limit data storage unit 42" or the like such that these ranges are normal ranges.

Moreover, the second soft the limit setting change data generation processing unit (program) 105 transmits the changed data to each of the infusion pumps 2a and so on in Fig. 1.

Thus, in ST22 to ST25, since the ranges of the "hard limit data" and the "soft limit data" are automatically changed to appropriate ranges on the basis of data, such as an actual alarm, it is possible to effectively execute an effective CQI process of medicine information such as the "hard limit data" and the "soft limit data" used in the infusion pumps 2a and so on.

Next, it proceeds to ST26. In ST26, the management server 10 transmits the "medicine-library-classified analysis data (for example, data in Fig. 9)" in the medicine-library-classified analysis data storage unit 45 in Fig. 4 to the medical office terminals 3a and so on in Fig. 1.

Further, the medical office terminals 3a and so on having received these items of data display these items of data on terminal side displays 4a and so on of the terminals.

Therefore, by visually perceiving the "medicine-library-classified analysis data" which is illustrated in Fig. 9 and displayed on the terminal side displays 4a and so on, the doctor or the like in the medical office can promptly understand the alert occurrence frequency of each administration rate.

Moreover, in the present embodiment, the ranges of the "soft limit" and the "hard limit" or the like are always automatically corrected and changed in an appropriate manner.

Therefore, the doctor or the like does not have to voluntarily correct the ranges of the "soft limit" and the "hard limit" or the like, and an extremely easy-to-use system is provided.

### Reference Signs List

1 pump CQI system
2a to 2e infusion pump
3a to 3c medical office terminal
4a to 4c terminal side display
10 management server
11 management server control unit
12 management server side display
13 management server side input device
14 management server side communication device
15 management server side timer equipment
16 data aggregation period determination processing unit (program)
17 medicine-classified data extraction processing unit (program)
18 administration-rate-classified solution transmission number data generation processing unit (program)
19 administration-rate-classified solution transmission number comparison alert occurrence frequency data generation processing unit (program)
20 medicine-library-classified analysis data generation processing unit (program)
21 standard administration rate change comment data generation processing unit (program)
22 first hard/soft limit change comment data generation processing unit (program)
23 second hard/soft limit change comment data generation processing unit (program)
40 various storage units
41 pump data storage unit
41a pump data
42 each-medicine soft/hard limit data storage unit
42a each-medicine soft/hard limit data
43 medicine-classified data storage unit
44 administration-rate-classified solution transmission number data storage unit
45 administration-rate-classified solution transmission number comparison alert occurrence frequency data storage unit
46 medicine-library-classified analysis data storage unit
47 standard administration rate change comment data storage unit
48 first hard/soft limit change comment data storage unit
49 second hard/soft limit change comment data storage unit
101 standard administration rate change data generation processing unit (program)
102 first hard limit setting change data generation processing unit (program)
103 second hard limit setting change data generation processing unit (program)
104 first soft limit setting change data generation processing unit (program)
105 second soft the limit setting change data generation processing unit (program)
120 pump control unit
121 pump body unit
122 occlusion detection device
123 alert (alarm) device
124 pump side timer equipment
125 pump side communication device
126 pump history data storage unit

## Claims

1. A medicine information management system (1) for continuous quality improvement comprising:
a management server (10) including various storage units (40) and a management server control unit (11);
wherein each of the various storage units (40) includes a medicine soft/hard limit data storage unit (42) which is a medicine limitation information storage unit for limiting a use of medicine information of the medicine in association with administration rate;
**characterized in that**
the management server (10) includes a management server side display (12) and terminal side displays (4a,4b,4c);
an administration-rate-classified solution transmission number data storage unit (44) for storing a solution transmission number of each administration rate, and an administration-rate-classified alert occurrence frequency data storage unit (45) for storing data of an alert occurrence frequency of each administration rate;
and the management server control unit (11) is configured to control:
storing an information on the number of doses that is actual information on the number of doses classified by administration rate associated with a subject of the medicine used in medical equipment into the administration-rate-classified solution transmission number data storage unit (44);
storing an alert information generated in a case where the medicine is actually used in the administration rate , in association with the medicine information and the administration rate into the administration-rate-classified alert occurrence frequency data storage unit (45) as a number of the alert;
displaying medicinal analysis information including the information of the number of doses classified by administration rate, the information of the number of the alert, and the medicine limitation information as associated in a graphical user interface on a display unit of a target side terminal,
a standard administration rate storage unit configured to store a standard administration rate that is administration rate serving as a standard of the medicine information,
wherein the standard administration rate of the medicine is included in the medicine analysis information as information associated with the administration-rate-classified solution transmission number data;
and further transmitting the following data to the terminal side displays (4a, 4b, 4c): comment data to change the standard administration rate from the current standard administration rate to an administration rate where the number of doses is the largest, comment data to change and expand the range of the hard limit where in the soft limit is greater compared to other administration rates,
comment data to change and reduce the range of the soft limit where the solution transmission number of the information on the number of doses associated with administration rate in the soft limit is greater than the others and the ratio of the number of the alert associated with the number of doses and administration rate is a low administration rate.

2. The medicine information management system (1) according to claim 1, further comprising a medicine limitation change suggestion information that suggests a change of the medicine limitation information is generated based on the medicine analysis information, and this medicine limitation change suggestion information is displayed on the display unit of the subject side terminal.

3. The medicine information management system (1) according to claim 1, further comprising a standard administration rate change suggestion information that suggests a change of the standard administration rate is generated based on the medicine analysis information, and this standard administration rate change suggestion information is displayed on the display unit of the subject side terminal.

4. The medicine information management system (1) according to claim 2, further comprising a standard administration rate change suggestion information that suggests a change of the standard administration rate is generated based on the medicine analysis information, and this standard administration rate change suggestion information is displayed on the display unit of the subject side terminal.

5. The medicine information management system (1) according to claim 1, wherein the medicine limitation information is changed based on the medicine analysis information.

6. The medicine information management system (1) according to claim 2, wherein the medicine limitation information is changed based on the medicine analysis information.

7. The medicine information management system (1) according to claim 3, wherein the medicine limitation information is changed based on the medicine analysis information.

8. The medicine information management system (1) according to claim 4, wherein the medicine limitation information is changed based on the medicine analysis information.

9. The medicine information management system (1) according to any one of claims 1 to 8, wherein the standard administration rate information is changed based on the medicine analysis information.

10. The medicine information management system (1) according to any of claims 1-9, wherein the management server control unit (11) is further configured to control:
displaying a graph illustrating the medicine analysis information,
wherein the graph, an administration rate data is arranged in the X axis direction, and the administration-rate-classified solution transmission number data and the administration-rate-classified alert occurrence frequency data are illustrated by a bar chart and by a line graph in the Y axis, further the medicine soft/hard limit data are displayed.

## Patentansprüche

1. Informationsverwaltungssystem (1) für Arzneimittel zur kontinuierlichen Qualitätsverbesserung, umfassend:
einen Verwaltungsserver (10), der verschiedene Speichereinheiten (40) und eine Verwaltungsserver-Steuerungseinheit (11) aufweist;
wobei jede der verschiedenen Speichereinheiten (40) eine Speichereinheit (42) für weiche/harte Arzneimittelbeschränkungsdaten umfasst, die eine Speichereinheit für Arzneimittelbeschränkungsinformationen ist, um eine Verwendung von Arzneimittelinformationen des Arzneimittels in Verbindung mit der Verabreichungsrate zu beschränken;
**dadurch gekennzeichnet, dass**
der Verwaltungsserver (10) eine verwaltungsserverseitige Anzeige (12) und endgeräteseitige Anzeigen (4a, 4b, 4c) umfasst;
eine Datenspeichereinheit (44) für Verabreichungsraten-klassifizierte Lösungsübermittlungsnummern zum Speichern einer Lösungsübermittlungsnummer jeder Verabreichungsrate, und eine Datenspeichereinheit (45) für eine Verabreichungsraten-klassifizierte Häufigkeit des Auftretens von Warnungen zum Speichern von Daten einer Häufigkeit des Auftretens von Warnungen für jede Verabreichungsrate;
und die Verwaltungsserver-Steuerungseinheit (11) so konfiguriert ist, dass sie steuert:
das Speichern einer Information über die Anzahl von Dosen, die eine tatsächliche Information über die Anzahl von Dosen ist, die durch die Verabreichungsrate klassifiziert sind, die mit einem Subjekt des Arzneimittels verbunden ist, das in einer medizinischen Ausrüstung verwendet wird, in der Datenspeichereinheit (44) für Verabreichungsraten-klassifizierte Lösungsübermittlungsnummern;
das Speichern einer Warninformation, die in einem Fall erzeugt wird, in dem das Arzneimittel tatsächlich in der Verabreichungsrate verwendet wird, in Verbindung mit der Arzneimittelinformation und der Verabreichungsrate in der Datenspeichereinheit (45) für eine Verabreichungsraten-klassifizierte Häufigkeit des Auftretens von Warnungen als eine Nummer der Warnung; das Anzeigen von medizinischen Analyseinformationen, welche die Informationen über die Anzahl der Verabreichungsraten-klassifizierten Dosen, die Informationen über die Nummer der Warnung und die Informationen über die Arzneimittelbeschränkung, die dazugehörig sind, umfasst, in einer grafischen Benutzeroberfläche auf einer Anzeigeeinheit eines zielseitigen Endgerätes,
eine Speichereinheit für Standard-Verabreichungsraten, die so konfiguriert ist, dass sie eine Standard-Verabreichungsrate speichert, die eine Verabreichungsrate ist, welche als Standard der Arzneimittelinformation dient, wobei die Standard-Verabreichungsrate des Arzneimittels in der Arzneimittel-Analyseinformation als Information enthalten ist, die mit den Verabreichungsraten-klassifizierten Lösungsübermittlungsnummer-Daten verbunden ist;
und ferner das Übertragen der folgenden Daten an die endgeräteseitigen Anzeigen (4a, 4b, 4c):
von Kommentardaten zum Ändern der Standard-Verabreichungsrate von der aktuellen Standard-Verabreichungsrate zu einer Verabreichungsrate, bei der die Anzahl der Dosen die größte ist,
von Kommentardaten zur Änderung und Erweiterung des Bereichs der harten Beschränkung, in dem die weiche Beschränkung im Vergleich zu anderen Verabreichungsraten größer ist,
von Kommentardaten zur Änderung und Verkleinerung des Bereichs der weichen Beschränkung, in dem die Lösungsübermittlungsnummer der Informationen über die Anzahl der Dosen im Zusammenhang mit der Verabreichungsrate in der weichen Beschränkung größer ist als die anderen und das Verhältnis der Nummer der Warnung, die mit der Anzahl der Dosen verbunden ist, und die Verabreichungsrate eine niedrige Verabreichungsrate ist.

2. Informationsverwaltungssystem (1) für Arzneimittel nach Anspruch 1, das ferner eine Information über einen Vorschlag zur Änderung der Arzneimittelbeschränkung umfasst, die eine Änderung der Arzneimittelbeschränkungsinformation vorschlägt, die auf der Grundlage der Arzneimittelanalyseinformation erzeugt wird, und diese Information über einen Vorschlag zur Änderung der Arzneimittelbeschränkung wird auf der Anzeigeeinheit des subjektseitigen Endgerätes angezeigt.

3. Informationsverwaltungssystem (1) für Arzneimittel nach Anspruch 1, das ferner eine Information zur Änderung der Standard-Verabreichungsrate umfasst, die eine Änderung der Standard-Verabreichungsrate vorschlägt, die auf der Grundlage der Arzneimittelanalyseinformation erzeugt wird, und diese Information zur Änderung der Standard-Verabreichungsrate wird auf der Anzeigeeinheit des subjektseitigen Endgerätes angezeigt.

4. Informationsverwaltungssystem (1) für Arzneimittel nach Anspruch 2, das ferner eine Information zur Änderung der Standard-Verabreichungsrate umfasst, die eine Änderung der Standard-Verabreichungsrate vorschlägt, die auf der Grundlage der Arzneimittelanalyseinformation erzeugt wird, und diese Information zur Änderung der Standard-Verabreichungsrate wird auf der Anzeigeeinheit des subjektseitigen Endgerätes angezeigt.

5. Informationsverwaltungssystem (1) für Arzneimittel nach Anspruch 1, wobei die Arzneimittelbeschränkungsinformationen auf der Grundlage der Arzneimittelanalyseinformationen geändert werden.

6. Informationsverwaltungssystem (1) für Arzneimittel nach Anspruch 2, wobei die Arzneimittelbeschränkungsinformationen auf der Grundlage der Arzneimittelanalyseinformationen geändert werden.

7. Informationsverwaltungssystem (1) für Arzneimittel nach Anspruch 3, wobei die Arzneimittelbeschränkungsinformationen auf der Grundlage der Arzneimittelanalyseinformationen geändert werden.

8. Informationsverwaltungssystem (1) für Arzneimittel nach Anspruch 4, wobei die Arzneimittelbeschränkungsinformationen auf der Grundlage der Arzneimittelanalyseinformationen geändert werden.

9. Informationsverwaltungssystem (1) für Arzneimittel nach einem der Ansprüche 1 bis 8, wobei die Arzneimittelbeschränkungsinformationen auf der Grundlage der Arzneimittelanalyseinformationen geändert werden.

10. Informationsverwaltungssystem (1) für Arzneimittel nach einem der Ansprüche 1 bis 9, wobei die Verwaltungsserver-Steuerungseinheit (11) ferner so konfiguriert ist, dass sie steuert:
das Anzeigen eines Diagramms, das die Arzneimittelanalyseinformationen veranschaulicht, wobei in dem Diagramm, in dem die Daten einer Verabreichungsrate in Richtung der X-Achse angeordnet sind und die Daten der Verabreichungsraten-klassifizierten Lösungsübermittlung und die Daten der Verabreichungsraten-klassifizierten Häufigkeit des Auftretens von Warnungen durch ein Balkendiagramm und ein Liniendiagramm in der Y-Achse veranschaulicht werden, ferner die Daten der weichen/harten Arzneimittelbeschränkungen angezeigt werden.

## Revendications

1. Système de gestion d'informations de médicament (1) pour une amélioration de qualité continue comprenant :
un serveur de gestion (10) incluant diverses unités de stockage (40) et une unité de commande de serveur de gestion (11) ;
dans lequel chacune des diverses unités de stockage (40) inclut une unité de stockage de données de limite souple/rigide de médicament (42) qui est une unité de stockage d'informations de limitation de médicament pour des informations de limitation d'utilisation de médicament du médicament en association avec un taux d'administration ;
**caractérisé en ce que**
le serveur de gestion (10) inclut un affichage côté serveur de gestion (12) et des affichages côté terminal (4a, 4b, 4c) ;
une unité de stockage de données de nombre de transmissions de solutions classées par taux d'administration (44) pour stocker un nombre de transmissions de solutions de chaque taux d'administration, et une unité de stockage de données de fréquence d'occurrence d'alerte classées par taux d'administration (45) pour stocker des données d'une fréquence d'occurrence d'alerte de chaque taux d'administration ;
et l'unité de commande de serveur de gestion (11) est configurée pour commander :
le stockage d'informations sur le nombre de doses constituant des informations réelles sur le nombre de doses classées par taux d'administration associé à un sujet du médicament utilisé dans un équipement médical dans l'unité de stockage de données de nombre de transmissions de solutions classées par taux d'administration (44) ;
le stockage d'informations d'alertes générées dans un cas dans lequel le médicament est effectivement utilisé au taux d'administration, en association avec les informations de médicament et le taux d'administration dans l'unité de stockage de données de fréquence d'occurrence d'alerte classées par taux d'administration (45) en tant que numéro de l'alerte ;
l'affichage d'informations d'analyse de médicament incluant les informations du nombre de doses classées par taux d'administration, les informations du numéro de l'alerte, et les informations de limitation de médicament qui sont associées dans une interface utilisateur graphique sur une unité d'affichage d'un terminal côté cible,
une unité de stockage de taux d'administration standard configurée pour stocker un taux d'administration standard qui est un taux d'administration servant de norme pour les informations de médicament,
dans lequel le taux d'administration standard du médicament est inclus dans les informations d'analyse de médicament en tant qu'informations associées aux données de nombre de transmissions de solutions classées par taux d'administration ;
et en outre la transmission des données suivantes aux affichages côté terminal (4a, 4b, 4c) :
des données de commentaire pour changer le taux d'administration standard du taux d'administration standard courant à un taux d'administration où le nombre de doses est le plus grand,
des données de commentaire pour changer et accroître la portée de la limite rigide dans lequel la limite souple est supérieure par rapport à d'autres taux d'administration,
des données de commentaire pour changer et réduire la portée de la limite souple où le nombre de transmissions de solutions des informations sur le nombre de doses associées au taux d'administration dans la limite souple est supérieur aux autres et le rapport du numéro de l'alerte associée au nombre de doses et au taux d'administration est un taux d'administration bas.

2. Système de gestion d'informations de médicament (1) selon la revendication 1, comprenant en outre des informations de suggestion de changement de limitation de médicament suggérant qu'un changement des informations de limitation de médicament soit généré sur la base des informations d'analyse de médicament, et ces informations de suggestion de changement de limitation de médicament sont affichées sur l'unité d'affichage du terminal côté sujet.

3. Système de gestion d'informations de médicament (1) selon la revendication 1, comprenant en outre des informations de suggestion de changement de taux d'administration standard suggérant qu'un changement du taux d'administration standard soit généré sur la base des informations d'analyse de médicament, et ces informations de suggestion de changement de taux d'administration standard sont affichées sur l'unité d'affichage du terminal côté sujet.

4. Système de gestion d'informations de médicament (1) selon la revendication 2, comprenant en outre des informations de suggestion de changement de taux d'administration standard suggérant qu'un changement du taux d'administration standard soit généré sur la base des informations d'analyse de médicament, et ces informations de suggestion de changement de taux d'administration standard sont affichées sur l'unité d'affichage du terminal côté sujet.

5. Système de gestion d'informations de médicament (1) selon la revendication 1, dans lequel les informations de limitation de médicament sont changées sur la base des informations d'analyse de médicament.

6. Système de gestion d'informations de médicament (1) selon la revendication 2, dans lequel les informations de limitation de médicament sont changées sur la base des informations d'analyse de médicament.

7. Système de gestion d'informations de médicament (1) selon la revendication 3, dans lequel les informations de limitation de médicament sont changées sur la base des informations d'analyse de médicament.

8. Système de gestion d'informations de médicament (1) selon la revendication 4, dans lequel les informations de limitation de médicament sont changées sur la base des informations d'analyse de médicament.

9. Système de gestion d'informations de médicament (1) selon l'une quelconque des revendications 1 à 8, dans lequel les informations de taux d'administration standard sont changées sur la base des informations d'analyse de médicament.

10. Système de gestion d'informations de médicament (1) selon l'une quelconque des revendications 1 à 9, dans lequel
l'unité de commande de serveur de gestion (11) est en outre configurée pour commander :
l'affichage d'un graphique illustrant les informations d'analyse de médicament,
dans lequel, dans le graphique, des données de taux d'administration sont agencées dans la direction de l'axe X, et les données de nombre de transmissions de solutions classées par taux d'administration et les données de fréquence d'occurrence d'alerte classées par taux d'administration sont illustrées par un diagramme à barres et par un graphique linéaire sur l'axe Y, en outre les données de limite souple/rigide de médicament sont affichées.
